(19) Europäisches Patentamt
European Patent Office
Office européen des brevets

(11) Publication number: **0 049 403**
**B1**

(12) **EUROPEAN PATENT SPECIFICATION**

(45) Date of publication of patent specification: **30.05.84**

(21) Application number: **81107374.1**

(22) Date of filing: **17.09.81**

(51) Int. Cl.[3]: **C 07 H 15/24,** C 07 C 50/36, A 61 K 31/70, A 61 K 31/12, C 07 D 317/26, C 07 C 69/18, C 07 C 69/16 // C07C49/733, C07D307/32, C07H13/08

(54) **Novel anthracycline derivative, process for production thereof, and pharmaceutical composition comprising said compound.**

(30) Priority: **22.09.80 JP 130645/80**

(43) Date of publication of application:
**14.04.82 Bulletin 82/15**

(45) Publication of the grant of the patent:
**30.05.84 Bulletin 84/22**

(84) Designated Contracting States:
**DE FR GB IT SE**

(56) References cited:
**GB-A-2 036 021**

**TETRAHEDRON LETTERS, vol. 21, no. 27, 1980, Pergamon Press A.V. RAMA RAO et al. "A simple synthesis of (+,-) 4-demethoxy-daunomycinone", pages 2661-2664**

(73) Proprietor: **ZAIDAN HOJIN BISEIBUTSU KAGAKU KENKYU KAI**
**14-23, Kamiosaki 3-chome**
**Shinagawa-ku Tokyo (JP)**

(72) Inventor: **Umezawa, Hamao**
**23, Toyotama Kita 4-chome**
**Nerima-ku Tokyo (JP)**
Inventor: **Takeuchi, Tomio**
**5-1-11, Higashigotanda**
**Shinagawa-ku Tokyo (JP)**
Inventor: **Naganawa, Hiroshi**
**3-17, Denenchofu Hon-cho**
**Ohta-ku Tokyo (JP)**
Inventor: **Tatsuta, Kuniaki**
**26-7, Matsunoki 2-chome**
**Suginami-ku Tokyo (JP)**

(74) Representative: **Kraus, Walter, Dr. et al**
**Patentanwälte Kraus & Weisert**
**Irmgardstrasse 15**
**D-8000 München 71 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 049 403 B1

**Description**

This invention relates to a novel anthracycline derivative, a process for the production thereof and a pharmaceutical composition comprising this compound.

Daunomycin (see US—A—3,616,242) and adriamycin (see US—A—3,590,028) obtained from fermentation broths of microorganisms of genus Actinomyces have been known as anthracycline-type antibiotics. These compounds have a broad antitumor spectrum against experimental tumors, and are widely used clinically as chemotherapeutic antitumor agents. Daunomycin and adriamycin show considerably strong antitumor actions, but these actions are never satisfactory. Numerous attempts have been made to create various analogous compounds having stronger antitumor actions by various means such as a fermentation method, a semisynthetic method, a totally synthetic method or a microorganism converting method. Some specific methods have already been proposed [see, for example, F. Arcmone, Topics in Antibiotic Chemistry, Vol. 2, pages 102 to 279, Ellis Horwood Limited; US—A—3,988,315 (aclacinomycins A and B)].

From Tetrahedron Letters, vol. 21 (1980, pages 2661—4), it is also known that the 4-demethoxyderivatives have improved anti-cancer properties GB—A—2,036,021 describes that 11-deoxy derivatives have comparable anti-cancer properties.

The object of the present invention is to provide a specific adriamycin derivative which has a higher activity than the corresponding compounds in the state of the art.

Subject matter of the present invention is a specific anthracycline derivative, namely 4-demethoxy-11-deoxyadriamycin having a (7S, 9S) configuration and of which amino sugar residue has a 1'$\alpha$ linkage, and its acid addition salts.

The inventive compound, 4-demethoxy-11-deoxyadriamycin having a (7S, 9S) configuration and of which amino sugar residue has a 1'$\alpha$ linkage, has the following formula

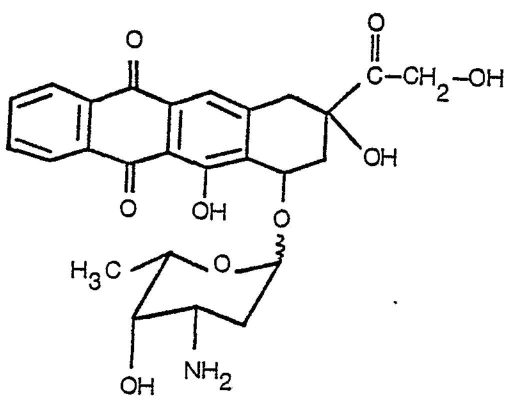

This novel compound or the acid addition salts of these have excellent antitumor activities as described hereinbelow, and are expected to be useful as antitumor agents.

The novel deoxyadiamycin derivative of the present invention may be prepared by a process which comprises hydrolizing a compound of the formula

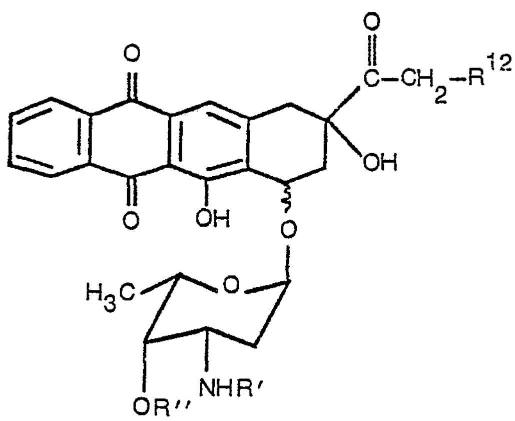

wherein $R^{12}$ represents a lower alkanoyloxy group, and R' and R'' respectively represent an amino-protecting group and a hydroxy-protecting group which can be easily eliminated by hydrolysis.

The acid addition salts of the compounds of formula (I-a-1) are preferably fromed with

pharmaceutically acceptable acids. Examples of such acids include inorganic acids such as hydrochloric acid, hydrobromic acid, sulfuric acid and phosphoric acid, and organic acids such as acetic acid, propionic acid, maleic acid, oleic acid, palmitic acid, citric acid, succinic acid, tartaric acid, fumaric acid, glutamic acid, pantothenic acid, laurylsulfonic acid, methanesulfonic acid and naphthalenesulfonic acid.

The compound of the present invention can be totally synthesized, for example, by a synthetic route shown by the following scheme.

Reaction Scheme

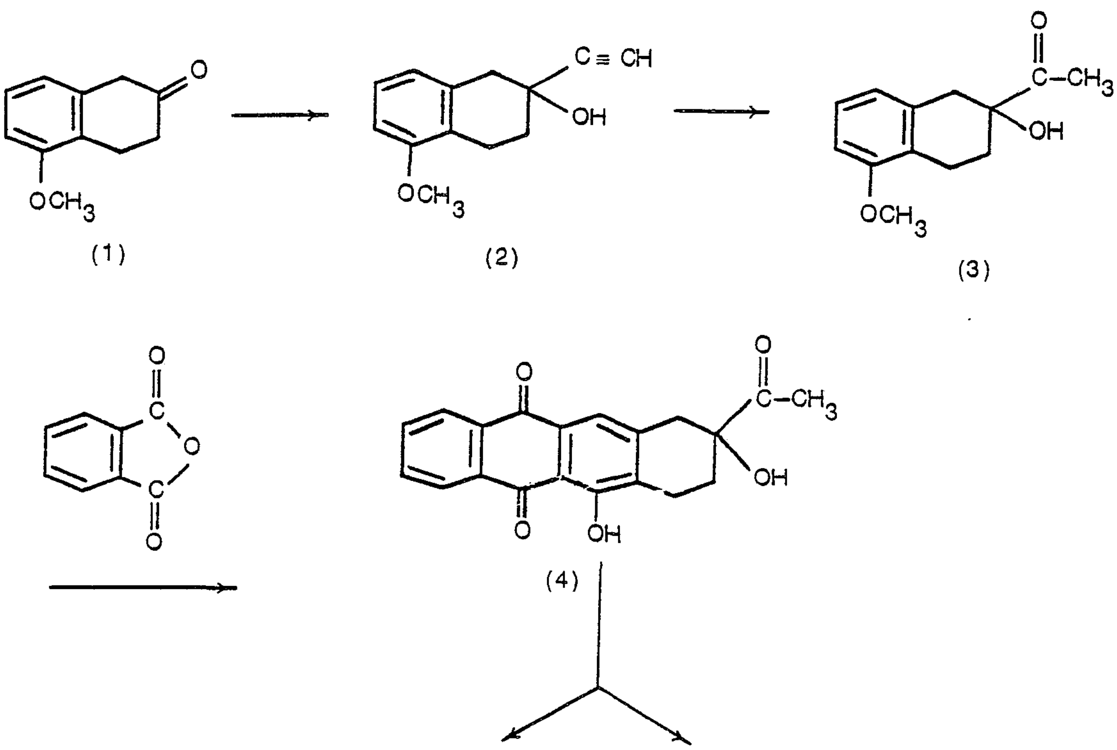

(1) (2) (3)

(4)

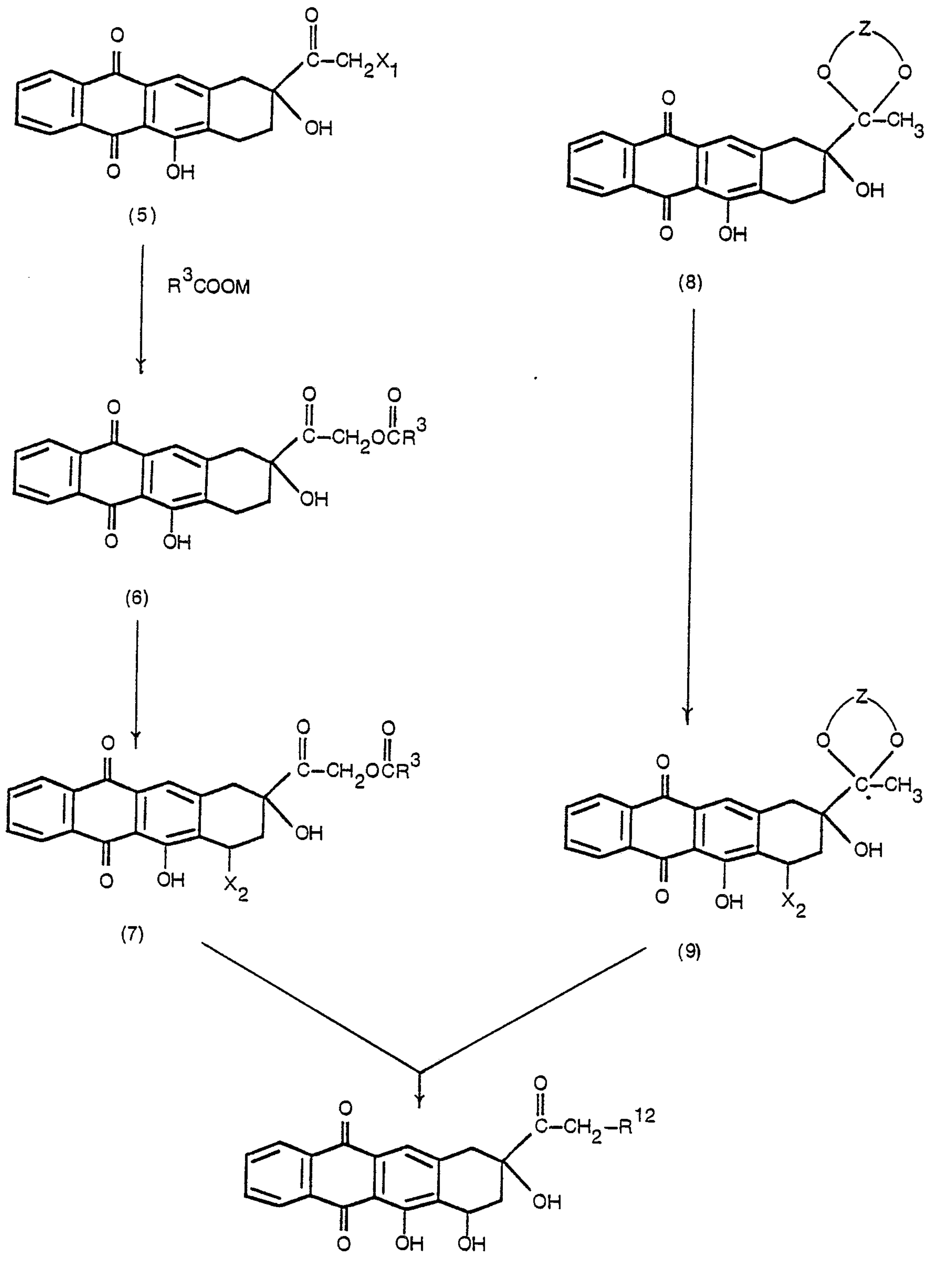


(5)

(6)

(7)

(8)

(9)

(I–b–1)

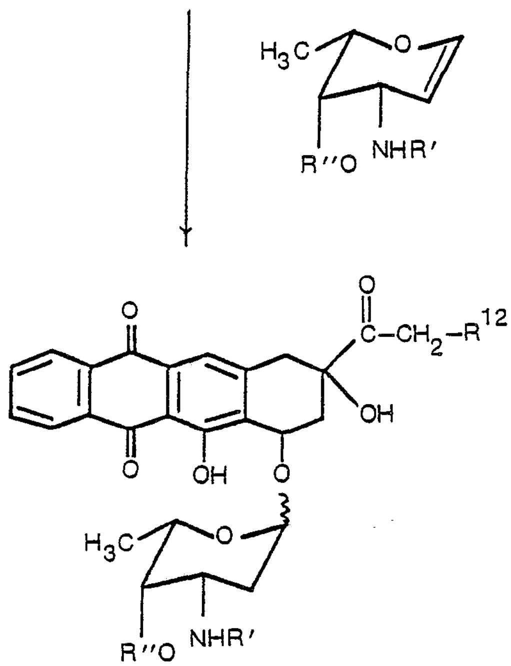

(III)

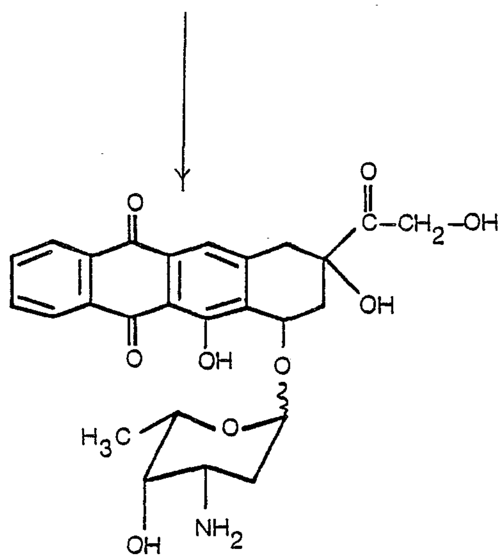

(I–a)

In the above reaction scheme, $R^{12}$ represents a hydrogen atom or a lower alkanoyloxy group; $R^3$ represents a lower alkyl group; $X_1$ and $X_2$ represent a halogen atom, especially a bromine atom; Z represents a ketal residue; M represents an alkali metal atom, an alkaline earth metal atom or an ammonium group; and R′ and R″ respectively represent an amino-protecting group and a hydroxyl-protecting group which can be easily eliminated by hydrolysis.

The unit reaction in each of the steps in the above reaction scheme is known *per se*, and can be practiced by known methods. The reactions in the individual steps are briefly described below.

(1) → (2)

In this step, 5-methoxy-2-tetralone of formula (1) is reacted with a Grignard reagent of the formula $(CH{\equiv}C)MgX_3$ (wherein $X_3$ represents a halogen atom, especially a bromine atom) to yield a tetraline derivative of formula (2). This reaction can be carried out by utilizing a known Grignard reaction, for example as shown in step A in Example 1 to be given hereinbelow.

(2) → (3)

In this step, hydrolysis of the tetraline derivative of formula (2) gives 2 - acetyl - 2 - hydroxy - 5 - methoxy - 1,2,3,4 - tetrahydronaphthalene. The hydrolysis can be carried out, for example, by treating the compound of formula (2) with sulfuric acid in the presence of mercuric oxide usually at room temperature.

The resulting compound of formula (3) can be isolated from the reaction mixture by an ordinary purifying method, for example by chromatography.

(3) → (4)

In this step, the compound of formula (3) prepared as above is reacted with phthalic anhydride to give 4 - demthoxy - 7,11 - dideoxydaunomycinone of formula (4). The above reaction can be performed in accordance with the Friedel-Crafts reaction. For example, this reaction can be carried out in the presence of a Lewis acid such as aluminum chloride, titanium tetrachloride or zinc chloride at a temperature of 50 to 250°C, especially 150 to 200°C, and can be completed in 1 to 30 minutes, preferably in 5 to 10 minutes. The phthalic anhydride is used in an amount of at least 1 mole, preferably 1.5 to 2 moles, per mole of the compound of formula (3). The Lewis acid is used in an amount of at least 8 equivalents, preferably 12 to 20 equivalents, per mole of the compound of formula (3). Preferably, the Friedel-Crafts reaction is carried out in the absence of a solvent at a relatively high temperature at which the starting materials substantially melt, usually at at least about 150°C, preferably 170 to 190°C. This effectively prevents the occurrence of the side-reaction mentioned below.

If the Friedel-Crafts reaction is carried out in the presence of an ordinary organic solvent or at a relatively low temperature (lower than about 140°C), a compound of the following formula

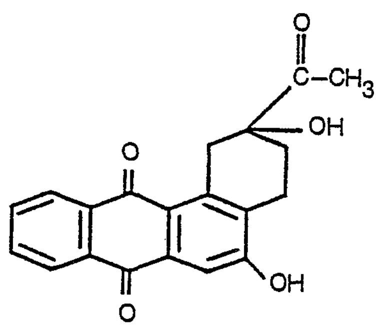

forms as a by-product in addition to the desired compound of formula (4), and decreases the yield of the compound of formula (4). It has been found that if the reaction is carried out in the absence of a solvent at a relatively high temperature, the compound of formula (4) can be obtained selectively in a high yield without the formation of such a by-product.

(4) → (5) → (6)

The reaction in this step can be carried out in a manner known *per se*, for example by the method described in US—A—3,803,124. Or the halogenation of the compound of formula (4) can be carried out by using an N-haloacid imide such as N-bromosuccinimide or N-chlorosuccinimide, especially the former, resulting in selective halogenation of the 14-position of the compound of formula (4). The halogenation with the N-haloacid imide can be carried out usually at a temperature of 0 to 50°C for about 2 to about 10 hours. The amount of the N-haloacid imide used is not critical. Generally, it is suitably used in an amount of at least 2 moles, preferably 8 top 16 moles, per mole of the compound of formula (4).

The acylation of the compound of formula (5) with a salt of an organic carboxylic acid having the formula $R^3COOM$ can be carried out at 0 to 80°C for about 5 to about 20 hours usually in an inert solvent, such as a ketone (e.g., acetone or methyl isobutyl ketone), or an ether (e.g., tetrahydrofuran or dioxane) either alone or as a mixture. The salt of an organic carboxylic acid can be used generally in an amount of 1.5 moles, preferably 2 to 4 moles, per mole of the compound of formula (5).

Examples of the salt of an organic carboxylic acid include sodium acetate, potassium acetate, ammonium acetate, sodium propionate, potassium propionate, ammonium propionate, sodium valerate and ammonium valerate.

(4) → (8)

The reaction in this stage is ketalization of the carbonyl group at the 13-position of the compound of formula (4), and can be carried out in a manner known *per se* using a carbonyl protecting reagent (ketalizing agent) known *per se*. For example, it can be performed by the action of a ketalizing agent of the formula HO—Z—OH on the compound of formula (4) in a suitable solvent in the presence of an

acid catalyst, for example an aromatic suflonic acid such as p-toluenesulfonic acid or benzene-sulfonic acid. The reaction temperature is not critical. Generally, the suitable temperature is from room temperature to the refluxing temperature of the reaction mixture, preferably from about 80°C to the refluxing temperature of the reaction mixture. The ketalizing agent is used in an amount of at least 1.5 moles, preferably from 5 to 15 moles, per mole of the compound of formula (4).

Specific examples of the ketal residue Z are $—CH_2CH_2—$,

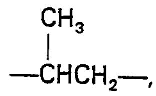, 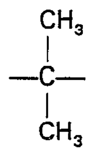 and 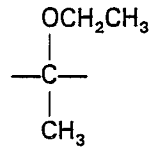

Hence, specific examples of the ketalizing agent include ethylene glycol, propylene glycol, 1,1-dimethoxypropane and triethoxymethane.

(6) → (7) and (8) → (9)

These steps involve halogenation of the 7-position of the compound of formula (6) or (8). The halogenation can be carried out by using known halogenating agents such as bromine, chlorine, N-bromosuccinimide and N-chlorosuccinimide in a customary manner. Bromination is especially preferred.

In a preferred embodiment, a solution containing bromine is added to the above compound in an aqueous medium in the presence of a free radical generator such as 2,2-azobisisobutyronitrile (to be abbreviated AIBN), and the reaction is carried out generally at 0 to 60°C, preferably at room temperature for 1 to 10 hours. The amount of the bromine solution is generally at least 1.5 moles, preferably 2 to 3 moles as bromine, per mole of the compound (6) or (8).

(7) or (9) → (I-b-1)

The reaction in this step is hydrolysis. Since the compound of formula (7) or (9) is unstable, when the above halogenation step is carried out in the presence of water, the hydrolysis of the compound (7) or (9) readily occurs to give the corresponding compound of formula (I-b-1). The above hydrolysis is carried out by a conventional method using an aqueous alkali solution under mild conditions, for example at a relatively low temperature of from room temperature to about 50°C using a weak alkali such as sodium hydrogen carbonate.

When the hydrolysis of the compound of formula (7) is performed under relatively strong hydrolyzing conditions, for example, by treating this compound with 10% potassium carbonate ($K_2CO_3$) at room temperature for 1 hour, a compound of formula (I-b-1) wherein $R^{12}$ is a hydroxyl group is formed.

The compound of formula (I-b-1) is generally obtained as a mixture of four stereoisomers having configurations (7S, 9S), (7R, 9R), (7R, 9S) and (7S, 9R).

A racemic mixture (7S, 9S; 7R, 9R) and a racemic mixture (7R, 9S; 7S, 9R) can be easily separated from the stereoisomeric mixture of the compounds of formula (I-b-1) in a customary manner, for example by a chromatographic technique.

The desired compound of formula (I-a-1) in this invention has a configuration (7S, 9S). Accordingly, the separated racemic mixture (7R, 9S; 7S, 9R) is epimerized to a racemic mixture (7S, 9S, 7R, 9R). As a result, the yield of the latter can be increased. The epimerization can be carried out, for example, by contacting the racemic mixture of the compound of formula (I-b-1) with an inorganic acid such as hydrochloric acid or perchloric acid in a water-soluble organic solvent such as acetone, tetrahydrofuran or dioxane at a temperature of from 10°C to the boiling point of the solvent.

(I-b-1) → (III)

In this step, the compound of formula (i-b-1) is reacted with a glycal of the formula

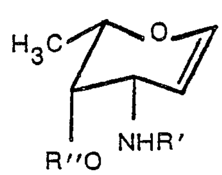

(10)

derived from daunosamine to give a glycoside of formula (III).

The amino-protecting group R′ in formula (10) may be those which can be easily eliminated by hydrolysis. Specific examples include lower alkanoyl groups such as acetyl, propionyl and trifluoroacetyl, aromatic carbonyl groups such as benzoyl and p-nitrobenzoyl, aralkyloxycarbonyl groups

such as benzyloxycarbonyl, and alkyloxycarbonyl groups such as t-butoxycarbonyl. The hydroxy-protecting groups R″ which can be easily eliminated by hydrolysis may be similar groups to those cited above for the amino-protecting group R′.

The glycosidation reaction can be carried out by a method known *per se* using an acid catalyst. Usually, it is carried out in an anhydrous organic solvent, for example benzene, toluene, tetrahydrofuran or dioxane, preferably in an aromatic hydrocarbon such as benzene or toluene in the presence of an acid catalyst, for example an aromatic sulfonic acid such as p-toluenesulfonic acid or benzenesulfonic acid or an alkylsulfonic acid such as methanesulfonic acid or butanesulfonic acid. The reaction temperature is advantageously 0 to 80°C, preferably 20 to 40°C. It is advantageous to use the compound of formula (10) in an amount of at least 1.5 moles, preferably 2 to 4 moles, per mole of the compound of formula (I-b-1).

The glycal of formula (10) can be produced by treating a daunosamine derivative having the 3-amino group and the 4-hydroxyl group protected with the above-exemplified groups, with a sulfonylating agent such as p-toluenesulfonyl chloride or benzylsulfonyl chloride in the presence of a base such as pyridine, dimethylaniline, morpholine or triethylamine in an organic solvent or in the absence of a solvent (see JP—A—27346/1979).

The glycoside of formula (III) according to the above glycoside-forming reaction is obtained usually as a mixture of an isomer whose amino sugar residue has a 1′α linkage (to be referred to as a 1′α isomer) and an isomer whose amino sugar residue has a 1′β-linkage (to be referred to as a 1′βisomer), and generally the proportion of the 1′α isomer is major.

Accordingly, when the racemic mixture (7S, 9S; 7R, 9R) of the compound of formula (I-b-1) is used as a starting material, the glycoside of formula (III) is a mixture of four steric isomers (7S, 9S, 1′α), (7S, 9S, 1′β), (7R, 9R, 1′α) and (7R, 9R, 1′β). These isomers can be separated individually by, for example, chromatography on silica gel or the like.

Isolation of these stereoisomers may be performed after the deprotecting reaction to be described hereinbelow.

(III) → (I-a)

This step is the elimination of the protective groups of the compound of formula (III). This deprotecting reaction is carried out by a known hydrolyzing method. For example, it can be effected by alkaline hydrolysis at a relatively low temperature of from about 0°C to room temperature in the presence of an alkali such as sodium carbonate, potassium carbonate. This hydrolysis results in the elimination of the protective groups R′ and R″ from the compound of formula (III). When $R^{12}$ is a lower alkanoyloxy group, the lower alkanoyl group is also eliminated depending upon the conditions of hydrolysis to give the compound of formula (I-a).

Anthracycline derivatives of the Formula I-b-1 and a process for their production are described and claimed in our copending divisional European application No. 83101145.7.

Thus, the anthracycline derivative of formula (I-a) can be obtained in a good yield.

The aglycone moiety shown in the present application is drawn in a planar structure, but is meant to include all configurations (7S, 9S), (7R, 9R), (7S, 9R) and (7R, 9S).

The compound of formula (III) produced by the above process in which $R^{12}$ represents a hydrogen atom can be converted to a compound of formula (III) in which $R^{12}$ is a lower alkanoyloxy group by subjecting it to the steps (4), (5) and (6) in this sequence.

The compound of formula (I-a) obtained as above can be converted to an acid addition salt by treating it with an inorganic or organic acid of the types exemplified hereinabove by methods known *per se.*

The compound of formula (I-a) has excellent antitumor activity, and that having the configuration (7S, 9S) which is the same as daunomycin or adriamycin produced by a fermentation method. The glycoside desirably has a 1′α-linkage.

The antitumor activity of the compound of formula (I-a) provided by this invention can be demonstrated by the following experiments.

(A) Activity of inhibiting growth, DNA synthesis and RNA synthesis of cultivated leukemic cells L1210 of mice.

The compound of this invention markedly inhibits the growth and nucleic acid synthesis of cultivated leukemic cells L1210 of mice. For example, L1210 cells were inoculated in a concentration of $5 \times 10^4$/ml in an RPMI 1640 culture medium containing 20% calf serum (Rosewall Memorial Park Institute 1640), and simultaneously the compound of this invention was added in a concentration of 0.1 and 0.5 μg/ml, respectively. Thus, the cells were cultivated in an incubator containing carbon dioxide gas at 37°C. The 50% growth inhibitory concentration with respect to a control group was determined.

Separately, the above cultivated cells L1210 were suspended in a concentration of $5 \times 10^5$/ml in an RPMI 1640 medium containing 10% of calf serum, and cultivated for 1 to 2 hours in an incubator containing carbon dioxide gas at 37°C. Then, the compound of this invention was added in various concentrations, and 15 minutes later, $^{14}C$-uridine (0.05 μCi/ml) and $^{14}C$-thymidine (0.05 μCi/ml) were added. Thus, the cells were cultivated for 60 minutes. A 10% aqueous solution of trichloroacetic acid

was added to the reaction liquor to stop the reaction and simultaneously precipitate an acid-insoluble matter. The acid-insoluble matter was washed three times with a 10-5% aqueous solution of trichloroacetic acid, and then dissolved in formic acid. The radioactivity of the acid-insoluble matter was measured. From the ratio of incorporated radiation to that of a control group, the concentrations which inhibited radiation by 10%, 50% and 90% respectively were measured. The results are shown in Table 1.

TABLE 1

Activity of the compounds of the invention to inhibit the growth, DNA synthesis and RNA synthesis of cultivated leukemic cells L1210 of mice

| | Compound | $IC_{50}$ (μg/ml) | | | | $IC_{10}$ (μg/mg) | | $IC_{90}$ (μg/mg) | |
|---|---|---|---|---|---|---|---|---|---|
| | | 1 day later | 2 days later | DNA | RNA | DNA | RNA | DNA | RNA |
| Known compounds | Daunomycin | 0.049 | 0.036 | 0.3 | 0.18 | 0.072 | 0.032 | 1.8 | 1.8 |
| | Adriamycin | 0.05 | 0.03 | 1.65 | 0.68 | 0.19 | 0.12 | 4.5 | 6.5 |
| | 4-Demethoxy-daunomycin | 0.01 | 0.005 | 0.08 | 0.14 | 0.007 | 0.017 | 1.2 | 1.4 |
| | 11-Deoxy-daunomycin | 0.05 | – | – | – | – | – | – | – |
| | 4-Demethoxy-adriamycin | 0.02 | 0.006 | 1.6 | 1.2 | 0.12 | 0.15 | 10 | 6.0 |
| | 11-Deoxy-adriamycin | 0.005 | – | – | – | – | – | – | – |
| Compound of the invention | 4-Demethoxy-11-deoxy-adriamycin (7S, 9S, 1'α) | 0.07 | 0.03 | 0.64 | 0.8 | 0.07 | 0.08 | 5.6 | 4.6 |

(B) Antitumor activity on CDFI mouse leukemia induced by leukemic cells L1210 of mice

Leukemic cells L1210 of mice were intraperitoneally transplanted in an amount of $1 \times 10^5$ per mouse in CDFI mice. Starting 24 hours after the transplantation, each of the compounds of the invention was intraperitoneally administered to the mice for 10 consecutive days. The survival rate (T/C, %) was calculated in comparison with a control group (to which physiological saline was administered). The results are shown in Table 2.

TABLE 2

Antitumor activity (T/C, %)

| Dosage (mg/kg/day, mouse) | Survival rate (T/C, %) | | | | | |
|---|---|---|---|---|---|---|
| | 5 | 2.5 | 1.25 | 0.6 | 0.3 | 0.15 |
| Daunomycin | Dead through toxicity | 138 | 191 | 145 | 132 | 118 |
| Adriamycin | 189 (*) | 351 | 272 | 239 | 147 | 130 |
| 4-Demethoxy-11-deoxyadriamycin (7S, 9S, 1'α) | — | 177* | 308 | 184 | 162 | 135 |

(*) Toxicity

As can be seen from the above experimental results, the compounds of formula (I-a) provided by the present invention, especially those of formula (I-a-1), exhibit excellent antitumor activity on leukemic cells L1210 and experimental animal tumors. The antitumor activity of 4 - demethoxy - 11 - deoxy - adriamycin (7S, 9S, 1'$\alpha$) stereoisomer is particularly noteworthy.

The compound of formula (I-a) provided by the invention is also characteristic in that it has antimicrobial activity. Table 3 summarizes the minimum growth inhibitory concentrations (MIC) of the compound of the invention against various bacteria on a nutrient agar medium.

TABLE 3

MIC on various bacteria

| Bacteria | M.I.C. ($\mu$g/ml) |
|---|---|
| | Compound of the present invention |
| Staphylococcus aureus FDA 209p | 100 |
| ,, ,, Smith | 12.5 |
| Bacillus subtilis PCI 219 | 12.5 |
| ,, ,, NRRB–558<br>( ,, ,, NRRB–558) | 12.5 |
| Bacillus cereus ATCC 10702 | 25 |
| Bacillus megatherium APF | 12.5 |
| Sarcina lutea PCI 1001 | 100 |
| Micrococcus flavus FDA 16. | 50 |
| Micrococcus lysodeikticus IFQ 3333 | 50 |
| Corynebacterium bovis 1810 | 100 |
| Klebsiella pneumoniae PCI 602 | > 100 |
| Escherichia coli NIHJ | > 100 |
| Salmonella typhi T–63 | > 100 |
| Shigella flexneri 46 JS 11811 | > 100 |
| Pseudomonas aeruginosa A3 | > 100 |
| Candida albicans 3147 | > 100 |
| Mycobacterium smegmatis ATCC 607 | 12.5 |

As stated hereinabove, the compound of formula (I-a) or pharmaceutically acceptable acid addition salts thereof, which are provided by the present invention, have superior antitumor activity, and can be used for the treatment of solid and ascitic tumor as anti-malignant tumor therapeutic agents.

In actual administration, the compound of the invention can be formulated into a pharmaceutical composition comprising a mixture of an amount, effective for antitumor activity, of the compound of formula (I-a) or its pharmaceutically acceptable acid addition salt and a compatible pharmaceutical carrier or diluent. Generally, the compound of formula (I-a) or its pharmaceutically acceptable acid addition salt can be administered parenterally as a solution in injectable distilled water or physiologically saline.

It is administered to animals other than man as an injecting preparation intraperitoneally, subcutaneously, intravascularly (intravenously or intraarterially), or topically; and to man as an injecting preparation intravascularly (intravenously or intraarterially) or topically. It can be administered successively or intermittently so that in view of the results of animal experiments and various situations, the total amount of it does not exceed a certain limit. The dosage may, of course, vary depending upon the route of administration, and various conditions of a subject to be treated, for example, age, body weight, sex, sensitivity, diet, administration time, administration method, drugs jointly used, and the severity of the disease. Suitable amounts and numbers of administrations under a given set of conditions should be determined by a physician's dosage determination test based on the above guidelines. Usually, the dosage will be 0.2 to 5 mg/kg body weight.

The active compound in accordance with this invention shows antibacterial activity against Gram-positive bacteria, and can be administered in the forms and dosages illustrated above as therapeutic agents for diseases induced by Gram-negative bacteria. The number of administrations, the dosage form, etc. may be determined by any one skilled in the art with the same care as above.

The following Examples show the production of the compounds of this invention more specifically.

Example 1

Production of 14-O-acetyl-4-demethoxy-11-deoxyadriamycinone:—

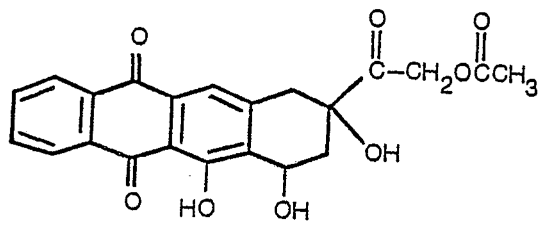

Step A

2-Ethinyl-2-hydroxy-5-methoxy-1,2,3,4-tetrahydronaphthalene:—

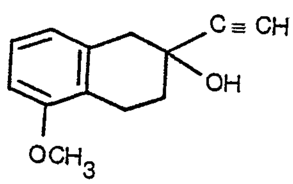

(2)

While acetylene was blown into 80 ml of anhydrous tetrahydrofuran; 36 ml of a 2M ether solution of ethylmagnesium bromide was added dropwise.

To the resulting solution was added 2.88 g of 5-methoxy-2-tetralone. After the reaction, the reaction mixture was poured into 500 ml of a saturated aqueous solution of ammonium chloride, and the mixture was extracted with 200 ml of carbon tetrachloride thrice. The extracts were washed with water, dried over sodium sulfate, and concentrated to dryness. The resulting brown oily product was chromatographed on a column of silica gel using benzene/ethyl acetate (25/l) as an eluent to give 1.6 g of the desired product as a brown oil.

NMRδ: 60MHz, $CDCl_3$

1.9 — 2.3 (m) —$CH_2$— at 3-position
2.40 (s) —C≡CH at 2-position
2.42 (s) —OH at 2-position
2.7 — 3.1 (m) —$CH_2$— at 4-position
3.05 — 3.2 (m) —$CH_2$— at 1-position
3.82 (s) —$OCH_3$ at 5-position
6.6 — 7.35 (m) at 6-, 7- and 8-positions

Step B

2-Acetyl-2-hydroxy-5-methoxy-1,2,3,4-tetrahydronaphthalene:—

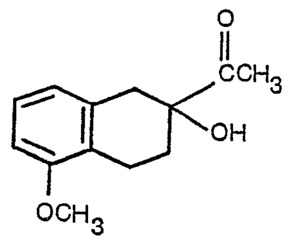

(3)

The compound of formula (2) was dissolved in 10 ml of carbon tetrachloride, and 10 ml of 1.5N sulfuric acid and 200 mg of mercuric oxide were added. They were reacted at room temperature for 27 hours. Water (40 ml) was added, and the reaction mixture was extracted with 50 ml of carbon tetrachloride four times. The extracts were washed with water, dried over sodium sulfate, and concentrated to dryness. The resulting oily product was chromatographed on a column of silica gel using benzene/ethyl acetate (20/1) as an eluent to give 840 mg of the desired product as colorless needles.

Melting point: 63—63.5°C

NMR $\delta$: 60 MHz, $CDCl_3$

1.8 — 2.1 (m) —$CH_2$— at 3-position
2.28 (s) $COCH_3$ at 2-position
2.5 — 3.5 (m) —$CH_2$— at 1- and 4-positions
3.45 (s) OH at 2-position
3.83 (s) $OCH_3$ at 5-position
6.6 — 7.3 (m) H at 6-, 7- and 8-positions

Step C

4-Demethoxy-7,11-dideoxydaunomycinone:—

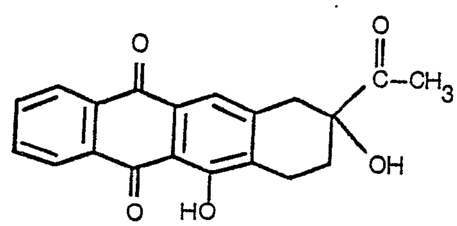

(4)

230 mg of the compound (3) obtained in step B, 230 mg of phthalic anhydride, 460 mg of sodium chloride and 2.3 g of aluminum chloride were well mixed, and melted by heating them to 180°C. In 1 to 2 minutes, the mixture became homogeneous, but was reacted further for 5 minutes.

The reaction mixture was treated with a saturated aqueous solution of oxalic acid, and extracted with 30 ml of chloroform five times. The extracts were combined, washed with water, dried over sodium sulfate, and concentrated to dryness. The product was chromatographed on a column of silica gel using benzene/ethyl acetate (20/1) as an eluent to give 110 mg of the desired product as a yellow solid.

Melting point: 208—214°C (decomp.)

NMR $\delta$: 60 MHz, DMSO

1.7 — 2.2 (m) —$CH_2$— at 8-position
2.30 (s) —$COCH_3$ at 9-position
2.6 — 3.1 (m) —$CH_2$— at 7- and 10-positions
5.60 (s) —OH at 9-position
7.38 (s) —H at 11-position
7.8 — 8.4 (m) —H at 1-, 2-, 3- and 4-positions
12.80 (s) —H at 6-position

Step D

14-O-Acetyl-4-demethoxy-7,11-dideoxyadriamycinone:—

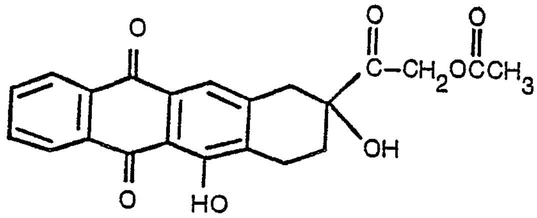

(6–a)

110 mg of the compound (4) obtained in step C was dissolved in 12 ml of tetrahydrofuran, and 200 mg of NBS was added four times at 1-hour intervals. The reaction was carried out at room temperature for a total period of 6 hours. The reaction mixture was poured into 200 ml of water, and extracted with 100 ml of carbon tetrachloride two times. The extracts were dried over sodium sulfate, and concentrated to dryness under reduced pressure. The product, without isolation and purification, was dissolved in 40 ml of acetone, and 200 mg of potassium acetate was added. The reaction was carried out at room temperature for 8 hours. After the reaction, the reaction mixture was poured into 50 ml of water, extracted with 26 ml of chloroform four times, dried over sodium sulfate, and then concentrated to dryness.

The product was chromatographed on a column of silica gel using benzene/ethyl acetate (40/1), benzene/ethyl acetate (30/1), benzene/ethyl acetate (20/1), and then benzene/ethyl acetate (15/1) as an eluent to give 83 mg of the title compound.

Melting point: 206—209°C

NMRδ: 100 MHz $CDCl_3$

2.0 — 2.3 (m) $—CH_2—$ at 8-position

2.20 (s) $—O\overset{\overset{O}{\|}}{C}—CH_3$ at 9-position

2.79 (s) —OH at 9-position

2.8 — 3.5 (m) $—CH_2—$ at 7- and 10-positions

5.11 (s) $—\overset{\overset{O}{\|}}{C}CH_2—O\overset{\overset{O}{\|}}{C}CH_3$

7.60 (s) —H at 11-position

7.75 — 7.9 (m) —H at 2- and 3-positions

8.2 — 8.4 (m) —H at 1- and 4-positions

13.03 (s) —OH at 6-position

Step E

14-O-Acetyl-4-demethoxy-11-deoxyadriamycinone:—

51 mg of the compound (6-a) was dissolved in 14 ml of chloroform, and 12 ml of water and 16 mg of azobisisobutyronitrile were added. To the solution was added 0.5 ml of 0.8% (W/V) bromine in carbon tetrachloride in seven portions at 1-hour intervals at room temperature, and the reaction was carried out for 9 hours. After the reaction, the unreacted bromine, carbon tetrachloride and chloroform were distilled off. The residue was dissolved in 40 ml of ethyl acetate, and 10 ml of a 0.1N aqueous solution of sodium hydrogen carbonate was added. The mixture was stirred at room temperature for a day and night. After the reaction, the reaction mixture was subjected to a separating procedure. The ethyl acetate layer was washed with water, dried over sodium sulfate, and concentrated to dryness. The dried product was chromatographed in the same way as in step A to give 34 mg of a racemic mixture (7S, 9S; 7R, 9R) of the title compound.

Melting point: 198—205°C (decomp.)

NMRδ: 100 MHz $CDCl_3$

2.0 — 2.6 (m) $—CH_2—$ at 8-position

2.19 (s) $O\overset{\overset{O}{\|}}{C}—CH_3$ at 9-position

3.28 —OH at 7-position
3.22 (AB) $-CH_2-$ at 10-position
4.69 (s) —OH at 9-position
5.25 (AB) $-\overset{O}{\overset{\|}{C}}-CH_2-O\overset{O}{\overset{\|}{C}}CH_3$
5.42 (m) —H at 7-position
7.68 (s) —H at 11-position
7.75 — 7.9 (m) —H at 2- and 3-positions
8.25 — 8.4 (m) —H at 1- and 4-positions
13.40 (s) —OH at 6-position

IR ($cm^{-1}$)

3450, 2950, 1750, 1730, 1670, 1630, 1590, 1480, 1420, 1385, 1360, 1330, 1285, 1270, 1245, 1215, 1160, 1110, 1100, 1065, 1060, 1040, 1030, 1010, 980, 960, 930, 910, 870, 840, 830, 820, 795, 770, 745, 715.

Example 2

Production of 4-demethoxy-11-deoxyadriamycin:—

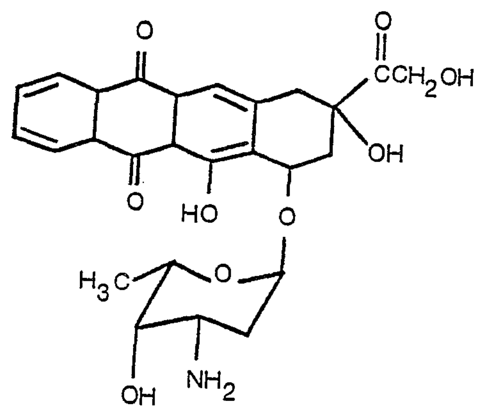

(I—a—1″)

Step A

4-O-p-nitrobenzoyl-1,2,3,6-tetradeoxy-3-trifluoroacetamide-L-lyxo-hex-1-enopyranose:—

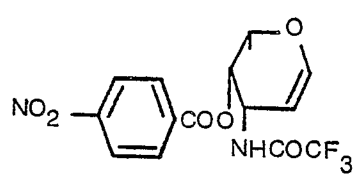

(1) 104 mg of N-trifluoroacetyldaunosamine was dissolved in anhydrous pyridine, and 300 mg of p-nitrobenzyl chloride was added. With ice cooling (about 8°C), the reaction was carried out for 22 hours. After the reaction, the reaction mixture was poured into 100 ml of ice water to destroy the excess of the reagent. The reaction mixture was then extracted with 30 ml of chloroform three times. In order to remove pyridine from the extracts, the extracts were subjected to a separating procedure using 50 ml of 1N hydrochloric acid. The chloroform layer was washed with water, dried over sodium sulfate, and concentrated.

(2) The concentrate (crude 1,4-bis-O-p-nitrobenzoyl-N-trifluoroacetyldaunosamine) was dissolved in 10 ml of acetone, and 5 ml of 4N hydrochloric acid was added. The reaction was carried out at room temperature for 10 hours to hydrolyze the p-nitrobenzoyl group partially. After the reaction, the acetone was removed by concentration and 15 ml of water was added. The mixture was extracted with 10 ml of chloroform three times. The extracts were subjected to a separating procedure using 10 ml of a 0.1N aqueous solution of sodium hydrogen carbonate. The chloroform layer was washed with water, dried over sodium sulfate, and concentrated.

(3) The concentrate (crude 4-O-p-nitrobenzoyl-N-trifluoroacetyldaunosamine) was dissolved in 15 ml of anhydrous pyridine, and 600 mg of p-toluenesulfonyl chloride was added. The reaction was

carried out at 80°C for 18 hours. The light brown reaction mixture was poured into 100 ml of ice water to destroy the excess of the reagent. The reaction mixture was then extracted with 30 ml of benzene four times. The extracts were washed with water, dried over sodium sulfate, and concentrated. The oily product was chromatographed on a column of silica gel using benzene/ethyl acetate (25/1) as an eluent to give 118 mg of the title compound.

Melting point: 144—148°C
$[\alpha]_D^{26}$: −100° (c: 0.5, acetone)
NMRδ: 100 MHz $CDCl_3$
1.32 (d) —$CH_3$ at 6-position
4.36 (broad q) H at 5-position
4.62 (td) H at 2-position
5.05 (m) H at 3-position
5.66 (broad d) H at 4-position
6.15 (broad d) —N*H*$COCF_3$
6.65 (dd) H at 1-position

8.15 — 8.4 (m) 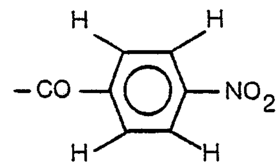

Step B
14-O-acetyl-4-demethoxy-4′-O-p-nitrobenzoyl-11-deoxy-3′-N-trifluoroacetyladriamycin:

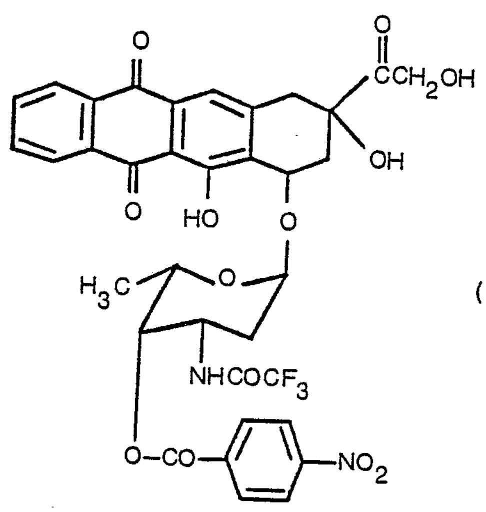

(III−b)

32 mg of the 14-O-acetyl-4-demethoxy-11-deoxy-adriamycinone (7S, 9S; 7R, 9R) obtained by the method of Example 1 and 120 mg of the glycal obtained in step A were dissolved in 12 ml of dichloromethane. A catalytic amount of p-toluene-sulfonic acid was added, and the reaction was carried out at room temperature for 75 hours. After the reaction, the reaction mixture was poured into 10 ml of a 0.01N aqueous solution of sodium hydrogen carbonate, and was extracted with 10 ml of dichloromethane two times. The extracts were washed with water, dried over sodium sulfate, and concentrated to dryness.

The product was chromatographed on a silica gel column using benzene/ethyl acetate (4/1) as an eluent, further chromatographed on a column of a crosslinked dextran gel (e.g., Sephadex® LH—20) using chloroform/acetone (1/2) as an eluent; and further chromatographed on a column of silica gel using benzene/ethyl acetate (4/1) as an eluent to isolate the individual stereoisomers of the title compound. The isolated crude products were again treated individually with Sephadex® LH—20 to give 10 mg of a (7S, 9S, 1′α) isomer (III-b-1) of the title compound and 10.7 mg of a (7R, 9R, 1′α) isomer (III-b-2) of the title compound.

(III-b-1)
Melting point: 154—157°C
$[\alpha]_D^{25}$: −125° (c: 0.2, acetone)
NMR δ: 100 MHz $CDCl_3$
1.31 (d) —$CH_3$ at 6′-position
1.9 — 2.7 (m) —$CH_2$—x2 at 8- and 2′-positions
2.22 (s) $OCOCH_3$
3.31 (AB) —$CH_2$— at 10-position
4.41 (s) —OH at 9-position
4.3 — 4.7 (m) —H at 3′-position
4.45 (broad q) —H at 5′-position
5.24 (AB) $-\overset{\overset{O}{\|}}{C}\mathit{CH_2}-OCOCH_3$
5.40 (m) —H at 7-position
5.50 (m) —H at 4′-position
5.70 (m) —H at 1′-position
6.30 (broad d) —N*H*—$COCF_3$
7.69 (s) —H at 11-position
7.75 — 7.95 (m) —H at 2- and 3-positions
8.2 — 8.45 (m) —H at 1- and 4-positions

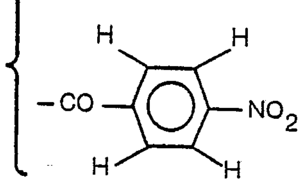

13.38 (s) —OH at 6-position

(III-b-2)
Melting point: 155—160°C
$[\alpha]_D^{25}$: −225° (c: 0.2 acetone)
NMR δ: 100 MHz $CDCl_3$
1.26 (d) —$CH_3$ at 6′-position
1.8—2.8 (m) —$CH_2$— at 8- and 2′-positions
2.20 (s) $OCOCH_3$
3.31 (broad s) —$CH_2$— at 10-position
4.4—4.85 (m) —H at 3′-position
4.72 (broad q) —H at 5′-position
4.62 (s) —OH at 9-position
5.21 (AB) —CO—*$CH_2$*—$OCOCH_3$
5.41 (m) —H at 4′-position
5.5—5.65 (m) —H at 7- and 1′-positions
6.52 (broad d) N*H*—$COCF_3$
7.63 (s) —H at 11-position
7.75 — 7.95 (m) —H at 2- and 3-positions
8.2 — 8.4 (m) —H at 1- and 4-positions

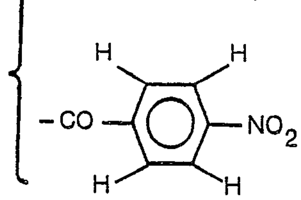

13.46 (s) —OH at 6-position

Step C

4-Demethoxy-11-deoxyadriamycin:—

(1) 12.3 mg of the compound (III-b-1) obtained in step A was dissolved in 6 ml of methanol, and 0.06 ml of a 10% aqueous solution of potassium carbonate was added. The reaction was carried out at 0°C for 3 hours. After the reaction, 30 ml of water was added, and extracted with 10 ml of chloroform three times. The extracts were washed with water, dried with sodium sulfate, and concentrated to dryness. The dried product was crude N-trifluoroacetyl-4-demethoxy-11-dioxyadriamycin (7S, 9S, 1′α).

(2) The dried crude product was dissolved in 3 ml of dichloromethane, and 1.2 ml of triethoxymethane and a catalytic amount of p-toluenesulfonic acid were added. The reaction was carried out at room temperature for 2.5 hours. After the reaction, 15 ml of a 0.1N aqueous solution of sodium hydrogen carbonate was added to neutralize the reaction mixture, followed by extraction with dichloromethane. The extract was dried over sodium sulfate, and concentrated to dryness.

(3) The resulting dried product was dissolved in 4 ml of methanol, and 1 ml of a 10% aqueous solution of potassium carbonate was added. With ice cooling (about 8°C), the reaction was carried out for 16 hours. After the reaction, 15 ml of water was added, and the reaction mixture was extracted with chloroform. The extract was treated with a 1% aqueous solution of acetic acid (when the orthoformate was eliminated). The acetic acid layer was adsorbed onto an adsorbent resin (e.g., Amberlite XAD—2, a tradename for a product of Rohm & Haas Co.), and eluted stepwise with acetone/0.0001N hydrochloric acid (20/80, acetone/0.0001N hydrochloric acid (30/70), and then acetone/0.0001N hydrochloric acid (40/60). After distilling off the acetone, the eluates were lyophilized to give 3.0 mg of the hydrochloride of a (7S, 9S, 1′α) isomer (I-a-1″-1) of 4-demethoxy-11-deoxyadriamycin as a yellow powder.

9.8 mg of the compound (III-b-2) obtained in step A was similarly treated to give 2.5 mg of the hydrochloride of a (7R, 9R, 1′α) isomer (I-a-1″-2) of 4-demethoxy-11-deoxyadriamycin.

Hydrochloride of (I-a-1″-1)
Melting point: 158—170° (decomp.)
$[\alpha]_D^{24}$: +75° (c: 0.1, water)
FDMS $MH^+$ 498
NMR δ: 100 MHz $D_2O$

1.80 (d) $—CH_3$ at 6′-position
2.4 — 2.9 (m) $—CH_2—$ at 8- and 2′-positions
3.45 (broad s) $—CH_2—$ at 10-position
4.1 — 4.4 (m) —H at 3′-position
4.33 (m) —H at 4′-position
—4.66 (broad q) —H at 5′-position
5.30 (s) $—\overset{\overset{O}{\|}}{C}—CH_2—OH$
5.94 (m) —H at 1′-position
7.53 (s) —H at 11-position
8.2 — 8.5 (m) —H at 1-, 2-, 3- and 4-positions

IR ($Cm^{-1}$)

3400, 2900, 1720, 1670, 1630, 1590, 1510, 1480, 1420, 1385, 1360, 1330, 1300, 1275, 1250, 1200, 1115, 1080, 1010, 980, 940, 910, 875, 825, 770, 715.

Hydrochloride of (I-a-1″-2)
Melting point: 145—155°C (decomp.)
$[\alpha]_D^{25}$: −125° (c: 0.1, water)
FDMS $MH^+$ 498
NMRδ: 100 MHz $D_2O$

1.73 (d) $—CH_3$ at 6′-position
2.3 — 3.2 (m) $—CH_2—$ at 8- and 2′-positions
3.50 (broad s) $—CH_2—$ at 10-position
4.1 — 4.3 (m) —H at 3′-position
4.32 (m) —H at 4′-position
4.84 (broad q) —H at 5′-position
5.33 (s) $—\overset{\overset{O}{\|}}{C}—CH_2—OH$
5.47 (m) —H at 7-position
5.91 (m) —H at 1′-position
7.48 (s) —H at 11-position
8.2 — 8.5 (m) —H at 1-, 2-, 3- and 4-positions

IR ($cm^{-1}$)

3450, 2900, 1720, 1670, 1630, 1590, 1480, 1425, 1390, 1365, 1330, 1300, 1280, 1255, 1195, 1110, 1075, 1010, 985, 935, 910, 830, 790, 770, 715.

**Claims**

1. 4-Demethoxy-11-deoxyadriamycin having a (7S, 9S) configuration and of which amino sugar residue has a 1'$\alpha$ linkage, or its acid addition salt.

2. A process for producing the compound of claim 1, which comprises hydrolizing a compound of the formula

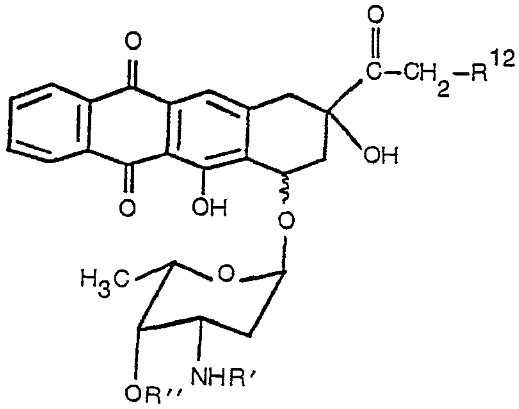

wherein $R^{12}$ represents a lower alkanoyloxy group, and R' and R'' respectively represent an amino-protecting group and a hydroxy-protecting group which can be easily eliminated by hydrolysis.

3. A pharmaceutical composition comprising

(1) 4-demethoxy-11-deoxyadriamycin having a (7S, 9S) configuration and of which amino sugar residue has a 1'$\alpha$ linkage, or its acid addition salt and

(2) a compatible carrier or diluent.

4. An antitumor agent comprising the compound of claim 1 or its pharmaceutically acceptable acid addition salt.

**Revendications**

1. 4-Déméthoxy-11-désoxyadriamycine ayant une configuration (7S, 9S) et dont le radical d'amino-sucre comporte une liaison 1'$\alpha$, et ses sels d'addition d'acides.

2. Procédé de préparation du composé selon la revendication 1, comprenant l'hydrolyse d'un composé de formule

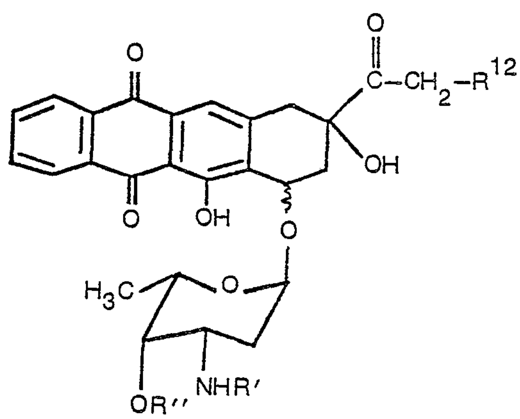

dans laquelle $R^{12}$ représente un groupe alcanoyloxy inférieur et R' et R'' représentent respectivement un groupe protégeant un groupe amino et une groupe protégeant un hydroxyle pouvant être facilement enlevés par hydrolyse.

3. Composition pharmaceutique comprenant:

(1) la 4-déméthoxy-11-désoxyadriamycine ayant une configuration (7S, 9S) et dont le radical d'amino-sucre comporte une liaison 1'$\alpha$, ou un sel d'addition d'acide de cell-ci, avec

(2) un véhicule ou diluant compatible.

4. Agent antitumoral comprenant le composé selon la revendication 1 ou un sel d'addition d'acide de celui-ci utilisable en pharmacie.

**Patentansprüche**

1. 4-Demethoxy-11-deoxyadriamycin mit einer (7S, 9S) Konfiguration von dem der Aminozuckerrest eine 1'$\alpha$ Verknüpfung aufweist, oder sein Säurreadditionssalz.

2. Verfahren zur Herstellung der Verbindung nach Anspruch 1, dadurch gekennzeichnet, daß man eine Verbindung der Formel

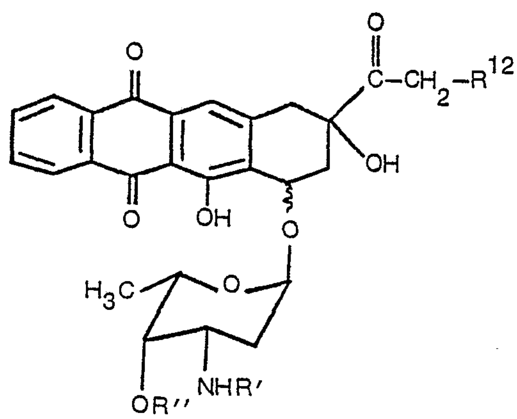

in der $R^{12}$ für eine Niedrigalkanoyloxygruppe steht, und R' bzw. R'' eine durch Hydrolyse leicht eleminierbare Aminoschutsgruppe und eine Hydroxyschutzgruppe bedeuten, hydrolysiert.

3. Arzneimittel dadurch gekennzeichnet, daß es

(1) 4-Demethoxy-11-deoxyadriamycin mit einer (7S, 9S) Konfiguration von dem der Aminozuckerrest eine 1'$\alpha$ Verknüpfung aufweist, oder sein Säureadditionssalz und

(2) einen verträglichen Träger oder Verdünnungsmittel enthält.

4. Antitumormittel dadurch gekennzeichnet, daß es die Verbindung nach Anspruch 1 oder ihr pharmazeutisch annehmbares Säureadditionssalz enthält.